# FASCICULE DE BREVET EUROPEEN

(11) **EP 2 082 684 B1**
(45) Date de publication et mention de la délivrance du brevet: **16.12.2009**
(21) Numéro de dépôt: 08291255.1
(22) Date de dépôt: 30.12.2008
(51) Int. Cl.: A61B 5/042, A61N 1/05, A61N 1/375, A61N 1/36, A61N 1/39, H03K 3/017, H04L 29/02

(54) **Dispositif médical implantable actif comprenant des moyens de communication bidirectionnelle entre en générateur et des capteurs ou actionneurs situés en extrémité de sonde**
Aktives medizinisches Implantat, das bidirektionelle Kommunikationsmittel zwischen einem Generator und Sensoren oder Stellgliedern umfasst, die sich am Ende einer Sonde befinden
Active implantable medical device comprising bi-directional communication means between a generator and sensors or actuators located on the end of the probe

(30) Priorité: 25.01.2008 FR 0800375
(43) Date de publication de la demande: 29.07.2009
(73) Titulaire: Ela Medical, 92541 Montrouge (FR)
(72) Inventeur: Dal Molin, Renzo, 92320 Châtillon (FR); Amara, Karima, 92330 Sceaux (FR)
(74) Mandataire: Dupuis-Latour, Dominique

(56) Documents cités:
- EP-A- 1 356 847
- WO-A-2006/029090
- US-A1- 2003 149 456

## Description

L'invention concerne, de façon générale, le domaine des "dispositifs médicaux actifs" tels que définis par la directive 93/42/CE du 14 juin 1993 du

Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les implants cochléaires, etc., ainsi que les dispositifs de mesure de pH ou encore d'impédance intracorporelle (telle que mesure d'impédance transpulmonaire ou d'impédance intracardiaque).

Ces dispositifs comportent un boîtier généralement désigné "générateur", raccordé électriquement et mécaniquement à une ou plusieurs "sondes" munie(s) d'électrodes destinées à venir en contact avec les tissus à stimuler et/ou sur lesquels on souhaite recueillir un signal électrique : myocarde, nerf, muscle, ... Dans le cas d'un dispositif de diagnostic et de thérapie cardiaque, ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

Un premier aspect du développement de ces appareils est la multiplication du nombre d'électrodes, notamment pour les appareils dits "multisite" qui permettent de choisir les sites de stimulation/détection optimisant le fonctionnement de l'appareil.

Ainsi, dans le cas particulier des dispositifs de resynchronisation ventriculaire (dispositifs dits CRT, *Cardiac Resynchronization Therapy*), on implante au patient un appareil muni d'électrodes permettant de stimuler l'un et l'autre ventricule. La stimulation du ventricule droit (et de l'oreillette droite) est opérée par une sonde endocavitaire classique, mais pour le ventricule gauche l'accès est plus complexe : la stimulation est généralement opérée au moyen d'une sonde introduite dans le sinus coronaire du ventricule droit puis poussée dans une veine coronaire sur l'épicarde, de façon que l'extrémité de la sonde vienne se placer face au ventricule gauche. Cette procédure est assez délicate, car le diamètre des vaisseaux coronaires se réduit avec l'avancement de la sonde, de sorte qu'il n'est pas toujours facile de trouver la position optimale lors de l'implantation. De plus, la proximité du nerf phrénique peut parfois conduire à des stimulations inappropriées. C'est pour pallier ces difficultés que les spécialistes ont été conduits à développer des sondes dites "multiélectrode", pourvues par exemple de dix électrodes et dont il est possible de choisir après implantation l'électrode de stimulation la plus efficace.

Pour gérer cette multiplicité d'électrodes, on a développé des systèmes de multiplexage permettant d'interfacer les diverses électrodes avec les deux conducteurs parcourant la sonde et reliés aux bornes du générateur. Le US 2003/0149456 A1 (Rottenberg et al.) décrit ainsi un générateur relié à une sonde multiélectrodes par deux conducteurs associés à un circuit multiplexeur/démultiplexeur. Ces deux conducteurs d'une part assurent le recueil des signaux de dépolarisation et l'envoi des impulsions de stimulation, et d'autre part délivrent au multiplexeur/démultiplexeur des signaux logiques permettant de contrôler des commutateurs de sélection d'une ou plusieurs électrodes de la sonde. Ces signaux assurent également la fourniture au circuit de multiplexage/démultiplexage et aux commutateurs de l'énergie nécessaire à leur fonctionnement. Les circuits de multiplexage/démultiplexage et les commutateurs sont de préférence situés en bout de sonde, donc à distance du générateur.

Un autre aspect du développement des appareils implantables est l'intégration à la sonde de capteurs divers, tout particulièrement de capteurs de pression sanguine ou d'accélération, notamment d'accélération endocardiaque (EA). Les signaux recueillis par ces capteurs permettent le pilotage de diverses fonctions du dispositif : en particulier, la mesure des pics d'accélération endocardiaque (PEA) donne une information représentative de l'état hémodynamique instantané du patient.

Les documents EP 0 515 319 A1, EP 0 582 162 A1 et EP 0 657 260 A1 (tous trois au nom de Sorin Biomedica Cardio SpA) décrivent ainsi des capteurs d'accélération endocardiaque montés à l'extrémité distale d'une sonde endocavitaire, introduite dans le myocarde et reliée au boîtier d'un stimulateur ou d'un défibrillateur.

La présence de tels capteurs en bout de sonde requiert une liaison spécifique pour la transmission des signaux depuis le capteur situé à l'extrémité distale de la sonde jusqu'au générateur connecté à l'extrémité opposée, proximale. Cette liaison propre au capteur vient se surajouter aux liaisons déjà existantes entre le générateur et les électrodes situées en bout de sonde, liaisons elles-mêmes assurées par des conducteurs spécifiques, avec ou sans multiplexage.

Enfin, un troisième aspect du développement récent des dispositifs médicaux implantables, notamment dans le domaine cardiaque, est la multiplication du nombre de sondes reliées à un même générateur. Il est ainsi courant, désormais, de voir des dispositifs comprenant trois sondes différentes, associées respectivement au ventricule droit, au ventricule gauche et à l'oreillette droite, chacune de ces sondes étant elle-même pourvue de plusieurs électrodes, et éventuellement d'un capteur en bout de sonde.

Le WO 2006/029090 A2 (Proteus Biomédical, Inc.) propose un système dans lequel un même bus bifilaire véhicule aussi bien des signaux numériques de sélection/configuration de satellites intégrés à la sonde, que les impulsions de stimulation et les signaux analogiques de recueil. La difficulté, et le risque, tient au fait que les signaux codés de sélection/configuration parviennent également au coeur via les électrodes de la sonde sous forme de salves d'impulsions, qu'il est nécessaire de calibrer très soigneusement pour ne pas risquer de déclencher une dépolarisation inopportune du tissu cardiaque.

L'un des buts de l'invention est de proposer un système capable de gérer en toute sécurité et de la façon la plus complète, la plus adaptable et la plus fiable ce type de situation, notamment pour des dispositifs mettant en oeuvre des sondes pourvues d'une multiplicité d'électrodes et/ou de capteurs, avec le cas échéant plusieurs sondes reliées à un même générateur.

L'invention propose un tel système qui soit en mesure de gérer globalement toutes les configurations de sondes et/ou électrodes et/ou capteurs susceptibles d'être associés à un même générateur, sans modification des composants matériels de ce générateur.

L'invention propose un dispositif médical implantable actif d'un type connu par exemple d'après le WO 2006/029090 A2 précité correspondant au préambule de la revendication 1, comprenant en outre les éléments originaux énoncés dans la partie caractéristique de cette revendication 1.

L'invention a également pour objet, considérés isolément, le générateur ainsi que la sonde d'un tel dispositif et, en tant que tel, un signal de commande mis en oeuvre au sein d'un tel dispositif.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 représente, de façon schématique, un dispositif médical comprenant un générateur et trois sondes destinées à être implantées dans trois cavités respectives du myocarde.

La Figure 2 illustre diverses formes d'impulsions transitant sur la liaison de communication bifilaire de l'une des sondes.

La Figure 3 illustre, sous forme de blocs fonctionnels, les différents circuits associés à la liaison entre le générateur et l'extrémité distale de la sonde, où se trouvent des électrodes, un actuateur et un capteur d'accélération.

La Figure 4 illustre, de façon plus détaillée, les divers composants du circuit de commande des actuateurs.

La Figure 5 est un organigramme exposant les différentes étapes du protocole de communication, vues du côté du générateur.

La Figure 6 est un organigramme exposant les différentes étapes du protocole de communication, vues du côté de la sonde.

On va maintenant décrire un exemple de réalisation de l'invention.

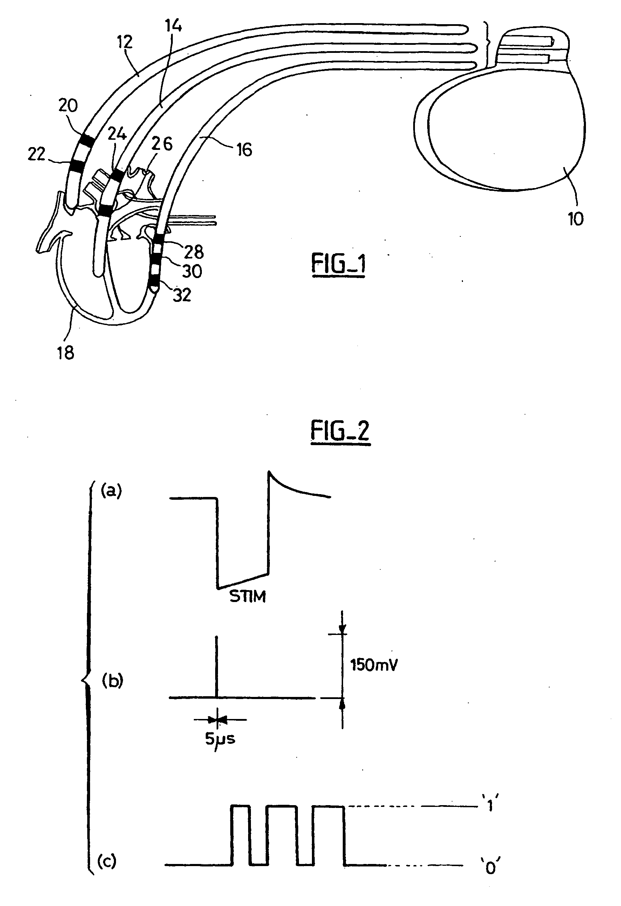

Sur la Figure 1, la référence 10 désigne le boîtier de générateur d'un dispositif de type défibrillateur, stimulateur ou resynchroniseur. On notera que cet exemple d'application n'est aucunement limitatif, et que l'invention peut s'appliquer à d'autres types d'implants tels que des dispositifs de détection/stimulation de nerfs, de muscles, etc.

Le générateur 10 est associé à trois sondes distinctes 12, 14 et 16 positionnées en divers emplacements du myocarde 18. La sonde 12 comporte à son extrémité distale deux capteurs ou actuateurs 20 et 22, par exemple un accéléromètre 20 et un actuateur 22 permettant la sélection d'une électrode de stimulation (on notera que le capteur et l'actuateur peuvent être situés dans un même module).

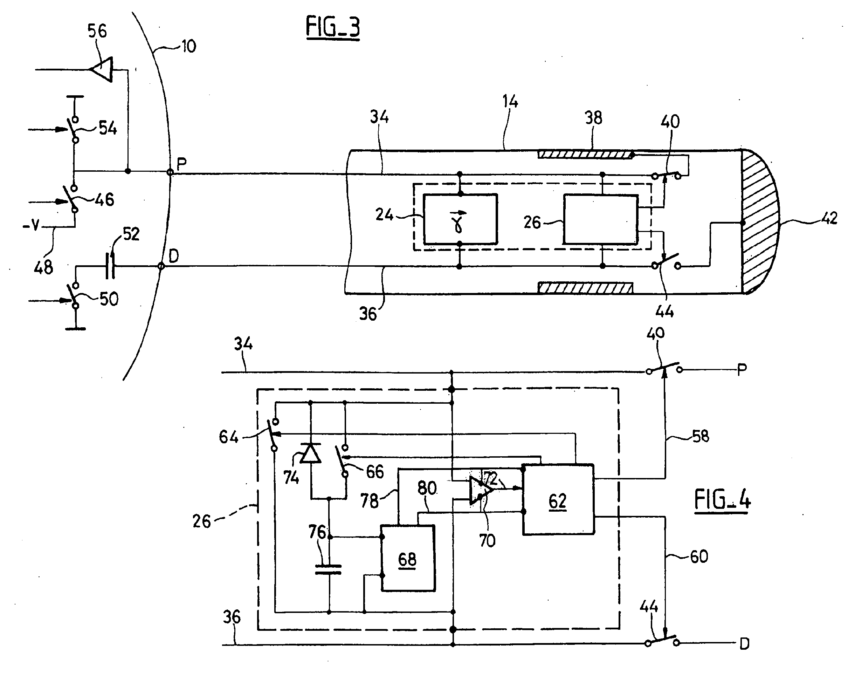

La sonde 14 intègre à son extrémité distale un capteur 24, par exemple un accéléromètre, et un actuateur 26 permettant la sélection d'une électrode de stimulation (ces éléments seront repris plus en détail en référence à la Figure 3). Enfin, la sonde 16 intègre à son extrémité distale trois capteurs ou actuateurs 28, 30, 32, par exemple un accéléromètre ou un capteur de pression 28 et deux actuateurs 30, 32 permettant la sélection de deux électrodes de stimulation respectives.

On a représenté de façon plus détaillée sur la Figure 3 l'extrémité de la sonde 14 (étant entendu que les deux autres sondes 12 et 16 sont configurées de façon semblable).

Cette sonde inclut deux conducteurs de liaison sous forme de deux microcâbles 34, 36 qui courent sur toute sa longueur et sont reliés au générateur 10. Ces deux conducteurs de liaison 34 et 36 seront désignés par la suite "microcâble proximal" 34 et "microcâble distal" 36, et les bornes correspondantes du générateur seront désignées "distale" et "proximale", par analogie avec le positionnement des deux électrodes d'une sonde bipolaire endocavitaire simple.

Les microcâbles 34 et 36 sont reliés à un capteur 24, par exemple un capteur d'accélération, ainsi qu'à un circuit de commande d'actuateur 26.

Par commodité, pour la simplicité de l'exemple, l'élément 24 est décrit sous forme d'un capteur, mais l'invention n'est pas limitée à ce type de composant : l'élément 24 peut être non seulement un capteur proprement dit (c'est-à-dire un transducteur de signal permettant de produire un signal électrique fonction des variations d'un paramètre physique), mais également un circuit électronique actif tel qu'amplificateur, filtre, ... associé ou non à un capteur disposé à proximité de ce circuit, ou encore un microsystème électromécanique (MEMS), ou encore de façon générale tout élément actif technologiquement intégrable à l'extrémité d'une sonde.

Le microcâble proximal 34 peut être relié à une électrode proximale 38 par l'intermédiaire d'un commutateur 40 contrôlé par le circuit 26, et le microcâble distal 36 peut être relié à une électrode distale 42 par l'intermédiaire d'un commutateur 44 contrôlé par ce même circuit 26 (dont la structure interne sera décrite ci-après plus en détail en référence à la Figure 4).

Les commutateurs 40 et 44 peuvent être, de manière en elle-même connue, réalisés sous forme de transistors MOS ou de microsystèmes électromécaniques (MEMS), composants technologiquement intégrables sur le substrat d'une puce qui peut être incorporée dans le corps de sonde. De tels composants sont par exemple décrits dans le document US 2004/0220650 A1.

Le générateur 10 est pourvu, outre les circuits de recueil/stimulation classiques (non représentés), de moyens permettant de lire ou d'écrire des données en provenance ou à destination du capteur 24 ou de l'actuateur 26, ou de tout autre capteur ou actuateur présent dans la sonde.

Pour générer un niveau logique '1' tel qu'illustré sur la Figure 2c (par convention, le niveau logique '1' correspond au niveau haut), le générateur commande la fermeture du commutateur 46 reliant le microcâble 34 à une source de tension continue 48 (par exemple -2 V) et du commutateur 50 reliant le microcâble 36 à la source de tension continue 48, ainsi que le commutateur 50 reliant le microcâble 36 au potentiel de référence via un condensateur 52 (le condensateur 52 est un condensateur d'isolement permettant d'éviter le passage de la composante continue ; il est prévu un système de commutation, non représenté, permettant de le décharger le moment venu). Simultanément, le générateur ouvre le commutateur 54 reliant le microcâble 34 au potentiel de référence (en variante, il est possible d'envoyer des impulsions positives sur le microcâble 36 par le commutateur 50 relié à une tension positive, en mettant le microcâble 34 à la masse par le commutateur 54).

Pour générer un niveau logique '0', le générateur ferme les commutateurs 50 et 54, et ouvre le commutateur 46. Pour la lecture d'un niveau logique '0' ou '1' (niveau bas ou haut, respectivement) les commutateurs 46 et 54 sont ouverts, et le commutateur 50 fermé : la lecture du niveau logique se fait alors par l'intermédiaire de l'amplificateur de lecture 56.

La structure interne du circuit de commande d'actuateur 26 est représentée plus en détail sur la Figure 4.

Ce circuit 26 assure non seulement la commande des interrupteurs 40 et 44, via des commandes 58, 60 produites par un circuit logique 62, mais également la génération de niveaux logiques '0' ou '1' à destination du générateur, de manière à permettre une communication bidirectionnelle de données non seulement dans le sens générateur → actuateur, mais également en retour dans le sens actuateur → générateur. Pour générer un niveau logique '0', le circuit 26 ferme un commutateur 64 mettant en liaison directe les deux microcâbles 34 et 36 ; l'interrupteur 66, dont le rôle sera exposé plus bas, est alors à l'état ouvert (dans la variante évoquée plus haut, le commutateur 50 serait fermé, et les commutateurs 54 et 46 ouverts). Pour générer un niveau logique '1', le circuit 26 ouvre le commutateur 64 et ferme le commutateur 66 permettant d'appliquer au microcâble 34 une tension fixe continue générée par un circuit 68. Les commutateurs 64 et 66 sont commandés par le circuit logique 62.

Pour mettre la sonde dans une configuration autorisant l'échange de données, le générateur produit une microimpulsion appliquée entre les microcâbles 34 et 36. Cette microimpulsion, représentée sur la Figure 2b, présente une durée, une polarité et/ou une amplitude spécifiques, discriminantes par rapport aux autres signaux susceptibles d'être présents sur les microcâbles 34 et 36, par exemple des signaux de stimulation (tels que ceux illustrés Figure 2a) ou d'injection de courant pour une mesure de bioimpédance. Cette microimpulsion a par exemple une durée de 5 µs et une amplitude de 150 mV. Un comparateur 70, dont les deux entrées sont reliées respectivement aux microcâbles 34 et 36, détecte cette forme particulière et en informe le circuit logique 62 par le signal 72. Le circuit logique 62 génère alors les signaux 58 et 60 qui commandent l'ouverture des commutateurs 40 et 44.

On notera que le comparateur 70 joue un double rôle : outre la détection d'une impulsion spécifique pour la commande des interrupteurs 40 et 44, il assure également la détection des niveaux logiques '0' ou '1' envoyés par le générateur à destination du circuit logique 62.

Pour fonctionner, le circuit 26 nécessite une alimentation. Celle-ci est apportée par les signaux logiques transitant sur les microcâbles 34 et 36, tels que ceux de la Figure 2c décrits plus haut. Pour assurer la continuité de cette alimentation, une diode 74 charge un condensateur série 76 lorsque la tension sur le microcâble 36 est supérieure à celle du microcâble 34. Le condensateur 76 conserve la charge et alimente le circuit 68, qui comprend un multiplicateur de tension permettant de générer les tensions 78 et 80 servant à alimenter le comparateur 58 et le circuit logique 62. La charge du condensateur 76 a lieu lorsque, côté générateur, les commutateurs 46 et 50 sont fermés et le commutateur 54 ouvert et que, côté actuateur, les commutateurs 64 et 66 sont ouverts. La décharge du condensateur 52 a lieu lorsque, côté générateur, les commutateurs 50 et 54 sont fermés et le commutateur 40 ouvert et, côté actuateur, le commutateur 64 est fermé et le commutateur 66 ouvert.

L'alimentation de tout autre capteur ou actuateur présent dans la sonde peut être réalisée de la même manière.

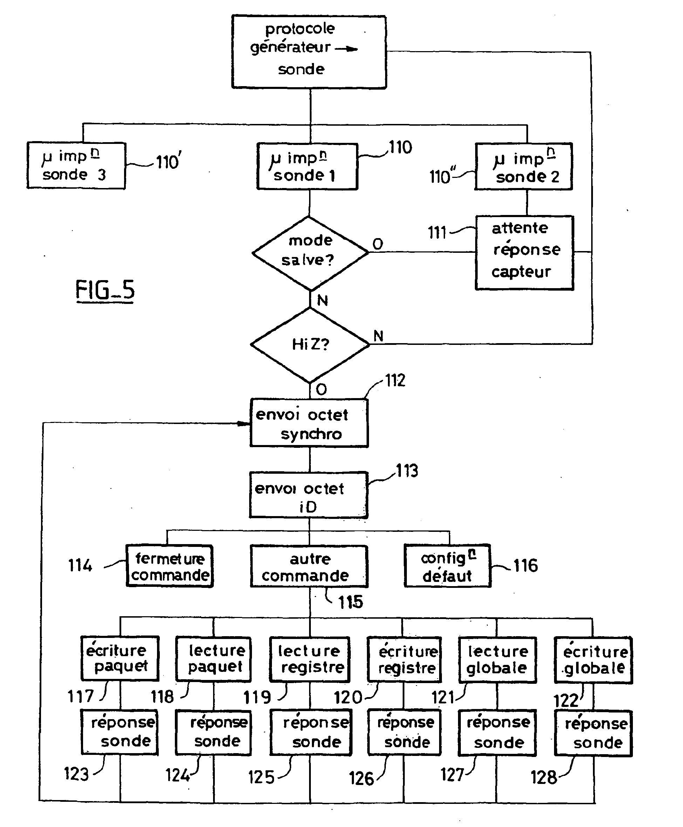

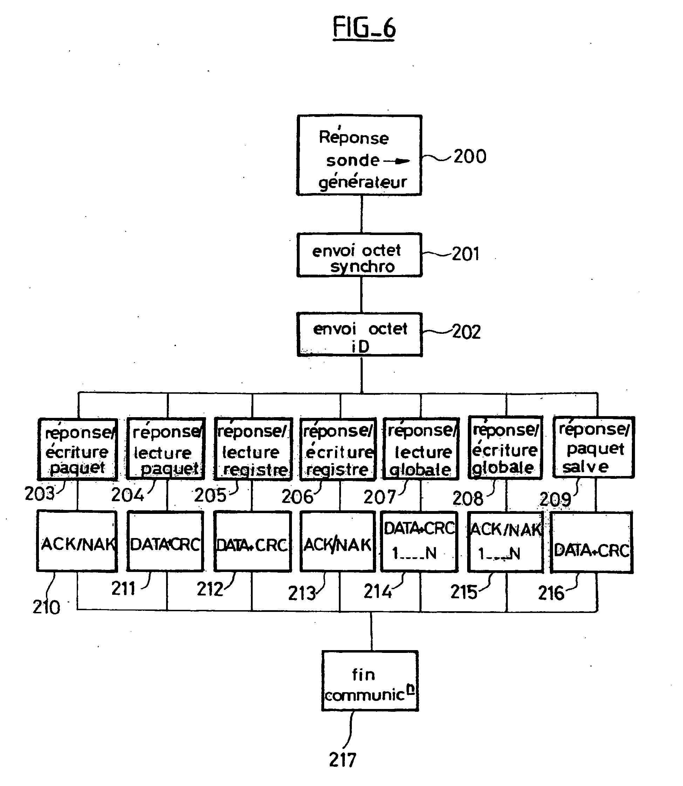

On va maintenant décrire, en référence aux Figures 5 et 6, le protocole de communication entre le générateur et les différents capteurs et actuateurs (ou autres éléments actifs semblables) intégrés aux différentes sondes reliées à ce générateur.

La Figure 5 illustre ce protocole vu depuis le générateur, tandis que la Figure 6 illustre ce même protocole vu depuis la sonde.

L'état initial est celui correspondant au bloc 100 (Figure 5), avec le générateur 10 de la Figure 1 relié à une pluralité de sondes 12, 14, 16 ... portant elles-mêmes une pluralité de capteurs et/ou actionneurs 20, 22 ... 30, 32 ... Chacun des capteurs ou actuateurs contient un registre de données numériques, destiné à être programmé, lu et/ou écrit à distance depuis le générateur 10.

Dans le protocole de communication, le générateur 10 est le maître. Il peut décider de programmer, lire ou écrire simultanément les registres d'une ou de plusieurs des sondes 12, 14 ou 16.

La sélection d'une sonde se fait par l'envoi d'une microimpulsion (blocs 110, 110' ou 110"), comme cela a été décrit précédemment et illustré en référence à la Figure 2b. La détection d'une telle microimpulsion par les capteurs et actuateurs de la sonde provoque la commande des commutateurs de déconnexion des électrodes associées aux microcâbles de la sonde. Dans l'exemple des Figures 3 et 4, cette microimpulsion est détectée par le circuit 70 qui, par l'intermédiaire du circuit logique 62, commande l'ouverture des commutateurs 40 et 44, ce qui a pour effet d'isoler les électrodes distale 42 et proximale 38 des microcâbles respectifs 36 et 34, qui ne sont donc plus reliés qu'au capteur 24 et à l'actuateur 26.

Avantageusement, le générateur peut fonctionner selon un mode optionnel dit "mode salve". Ceci signifie que par exemple toutes les 8 ms le capteur 24 envoie au générateur les données enregistrées accumulées au cours des dernières 8 ms. Si l'on se trouve dans un tel cas, alors le générateur attend une réponse du capteur de la sonde (bloc 111), cette réponse correspondant à la séquence de blocs 201, 209, 216 et 217 (Figure 6) avec successivement : envoi d'un octet de synchronisation (bloc 201) ; envoi d'un octet d'identification du registre dans lequel sont contenues les données transmises par le capteur en question (bloc 202). ; envoi d'un code indiquant que la réponse est une réponse de type paquet de données, spécifique du mode salve (bloc 209) ; envoi des données proprement dites et d'un code de vérification et de correction d'erreur (code CRC) permettant de sécuriser la transmission (bloc 216) ; achèvement de la communication (bloc 217).

On notera que, ici et dans la suite de la description détaillée, les commandes peuvent ne pas comporter de mot de synchronisation, par exemple si l'on utilise une modulation en largeur d'impulsions dont la durée est suffisamment importante pour tolérer des horloges désynchronisées, et dont la largeur détermine le niveau logique. Le mot de synchronisation peut par ailleurs inclure un préambule codé qui permet de tester la synchronisation des horloges.

D'autre part, les mots peuvent être codés en "Manchester".

Enfin, le mot d'identification permet d'identifier non seulement le registre, mais également le transducteur et la sonde : l'identification de la sonde est en effet nécessaire pour éviter, par diaphonie, de programmer une autre sonde munie d'actuateurs.

Dans le cas où le générateur n'est pas en mode salve, il vérifie par une mesure d'impédance (Figure 5) que les capteurs et actuateurs sont bien déconnectés des électrodes. Cette mesure est opérée, de manière classique, par injection d'un courant et mesure de la tension résultante entre les deux microcâbles. Si, par une valeur d'impédance élevée, cette mesure confirme la déconnexion effective des électrodes, alors le générateur envoie sur la liaison bifilaire un octet de synchronisation (bloc 112) afin de permettre la synchronisation des horloges du générateur avec les actuateurs et capteurs de la sonde sélectionnée.

Le générateur envoie ensuite un octet d'identification (bloc 113), suivi par l'une des trois instructions suivantes :
- interruption de la communication et fermeture des commutateurs de liaison aux électrodes, avec envoi d'un code CRC pour sécuriser la transmission (bloc 114) ;
- retour de tous les actuateurs et capteurs à une configuration par défaut, avec un code CRC pour sécuriser la transmission (bloc 116) ;
- autre commande à venir (bloc 115).

Peuvent suivre les commandes suivantes :
- écriture d'un paquet d'octets à partir d'une adresse donnée sur l'un des actuateurs ou capteurs d'une sonde (bloc 117) ;
- lecture d'un paquet d'octets à partir d'une adresse donnée sur l'un des actuateurs ou capteurs d'une sonde (bloc 118) ;
- lecture d'un registre à une adresse donnée sur l'un des actuateurs ou capteurs d'une sonde (bloc 119) ;
- écriture d'un registre à une adresse donnée sur l'un des actuateurs ou capteurs d'une sonde (bloc 120) ;
- lecture d'un registre à une adresse donnée sur tous les actuateurs et capteurs d'une même sonde (bloc 121);
- écriture d'un registre à une adresse donnée sur tous les actuateurs et capteurs d'une même sonde (bloc 122).

Toutes ces commandes sont suivies d'un code CRC permettant de sécuriser la transmission.

Le générateur se met alors en attente de la réponse de la sonde (blocs 123 à 128, faisant respectivement suite aux blocs 117 à 122).

La réponse, côté sonde, aux commandes 117 à 122 est schématisée par les blocs 203 à 207 sur la Figure 6.

La réponse de la sonde peut prendre selon les cas plusieurs formes (blocs 210 à 215, respectivement) :
- ACK : code indiquant une réception correcte de l'ordre émis par le générateur ;
- NAK : code indiquant que cette réception est erronée ;
- DATA + CRC : envoi des données demandées, suivi d'un code de contrôle CRC ;
- ACK/NAK 1 ... N : chaque actuateur ou capteur de la sonde répond séquentiellement par un ACK/NAK, dans un ordre donné ;
- DATA + CRC 1 ... N : chaque actuateur ou capteur de la sonde répond séquentiellement en fournissant les données demandées DATA, suivi d'un code de contrôle CRC associé, dans un ordre donné.

La communication prend ensuite fin (bloc 217). En tout état de cause, si une microimpulsion, produite par le générateur pour ouvrir les commutateurs des actuateurs, n'est pas suivie dans un délai donné (par exemple 100 µs) par un autre signal reçu du générateur, les commutateurs sont refermés pour permettre un retour à la configuration d'origine, de manière à pouvoir assurer avec certitude le recueil des signaux de dépolarisation cardiaque et/ou l'application de signaux de stimulation ou de défibrillation.

## Revendications

1. Un dispositif médical implantable actif, comprenant :
- un générateur (10), comportant :
des moyens d'analyse de signaux physiologiques et/ou de production d'impulsions de stimulation ; et
des moyens d'émission et de réception de données numériques, et
- au moins une sonde (12, 14, 16), reliée côté proximal au générateur et comportant, côté distal :
au moins une électrode (38, 42) apte à venir en contact avec des tissus environnants, pour appliquer lesdites impulsions de stimulation et/ou capter lesdits signaux physiologiques ;
une liaison bifilaire (34, 36) apte à relier l'(les) électrode(s) au générateur ; et
au moins un transducteur (24, 26) de type capteur ou actuateur ; et
une liaison de communication de données numériques, apte à coupler ledit au moins un transducteur au générateur par l'intermédiaire de la liaison bifilaire ; dans lequel :
- les moyens d'émission et de réception de données numériques du générateur comprennent des moyens (46, 48, 50, 54) pour produire des commandes à destination du transducteur,
- le transducteur comprend des moyens (62, 64, 66, 70) pour recevoir, décoder et exécuter lesdites commandes, ainsi que des moyens (62, 64, 66, 68) pour émettre des informations en réponse à la réception d'une commande spécifique parmi les commandes produites par le générateur, et
- les moyens d'émission et de réception de données numériques du générateur comprennent des moyens (56) pour recevoir et décoder lesdites informations, dispositif **caractérisé en ce que** :
- le générateur comprend en outre des moyens pour produire une microimpulsion de pilotage préalablement à l'envoi desdites commandes à destination du transducteur, cette microimpulsion présentant au moins une caractéristique de forme propre, distinctive par rapport aux impulsions de stimulation ; et
- la sonde comprend en outre côté distal :
des moyens interrupteurs commandés (40, 44), sélectivement interposés entre l'(les) électrode(s) (38, 42) et les conducteurs respectifs (34, 36) de la liaison bifilaire (34, 36) ; et
un circuit de pilotage (62, 70) couplé à la liaison bifilaire (34, 36), apte à :
i) reconnaître ladite caractéristique de forme propre parmi les impulsions détectées sur la liaison bifilaire, de manière à discriminer entre impulsions de stimulation et microimpulsion de pilotage ; et
ii) sur détection d'une microimpulsion de pilotage, commander l'ouverture des moyens interrupteurs (40, 44) de manière à isoler l'(les) électrode(s) (38, 42) de la liaison bifilaire (34, 36), celle-ci n'étant plus reliée qu'au transducteur (24, 26).

2. Le dispositif de la revendication 1, dans lequel ladite caractéristique de forme propre à la microimpulsion de pilotage est un caractéristique du groupe formé par : la durée de la microimpulsion ; la polarité de la microimpulsion ; l'amplitude de la microimpulsion ; et les combinaisons de ces caractéristiques.

3. Le dispositif de la revendication 1, dans lequel le circuit de pilotage comprend en outre des moyens (74, 76) pour produire une tension d'alimentation à partir des impulsions détectées sur la liaison bifilaire.

4. Le dispositif de la revendication 1, dans lequel lesdites commandes sont des commandes du groupe formé par : l'envoi d'un mot de synchronisation ; l'envoi d'un mot d'identification du transducteur destinataire ; la fermeture de la communication ; le retour à une configuration par défaut du transducteur ; l'écriture de données dans une mémoire du transducteur ; et la lecture de données depuis une mémoire du transducteur.

5. Le dispositif de la revendication 1, dans lequel lesdites informations sont des informations du groupe formé par : un mot de synchronisation ; un mot d'identification du transducteur émetteur et/ou récepteur ; des données contenues dans une mémoire du transducteur ; un accusé de réception suite à une action exécutée sur commande du générateur ; et un code de détection et de correction d'erreur.

6. Un générateur pour un dispositif médical implantable actif selon l'une des revendications 1 à 5, ce générateur (10) comprenant :
- des moyens de liaison à une sonde (12, 14, 16) apte à être reliée côté proximal au générateur, avec une liaison bifilaire (34, 36) apte à relier au générateur au moins une électrode (38, 42) de la sonde, et une liaison de communication de données numériques, apte à coupler au moins un transducteur (24, 26) de la sonde au générateur par l'intermédiaire de la liaison bifilaire ;
- des moyens d'analyse de signaux physiologiques et/ou de production d'impulsions de stimulation ; et
- des moyens d'émission et de réception de données numériques, comprenant :
des moyens (46, 48, 50, 54) pour produire des commandes à destination du transducteur ; et
des moyens (56) pour recevoir et décoder des informations reçues du transducteur en réponse à la réception d'une commande spécifique parmi lesdites commandes ;
générateur dans lequel lesdits moyens d'émission et de réception de données numériques comprennent en outre :
des moyens pour produire une microimpulsion de pilotage préalablement à l'envoi desdites commandes à destination du transducteur, cette microimpulsion présentant au moins une caractéristique de forme propre, distinctive par rapport aux impulsions de stimulation.

7. Une sonde pour un dispositif médical implantable actif selon l'une des revendications 1 à 5, cette sonde (12, 14, 16) comprenant :
côté distal, au moins une électrode (38, 42) apte à venir en contact avec des tissus environnants, pour appliquer des impulsions de stimulation et/ou capter des signaux physiologiques ; et au moins un transducteur (24, 26) de type capteur ou actuateur ;
- côté proximal, des moyens de liaison à un générateur (10) apte à être relié à la sonde, avec une liaison bifilaire (34, 36) apte à relier l'(les) électrode(s) au générateur, et une liaison de communication de données numériques apte à coupler le transducteur au générateur par l'intermédiaire de la liaison bifilaire,
- des moyens (62, 64, 66, 70) pour recevoir, décoder et exécuter des commandes produites par le générateur ; et
- des moyens (62, 64, 66, 68) d'émission d'informations en réponse à la réception d'une commande spécifique parmi lesdites commandes. sonde **caractérisée en ce qu'**elle comprend en outre, côté distal :
- des moyens interrupteurs commandés (40, 44), sélectivement interposés entre l'(les) électrode(s) (38, 42) et les conducteurs respectifs (34, 36) de la liaison bifilaire (34, 36) ; et
- un circuit de pilotage (62, 70) couplé à la liaison bifilaire (34, 36), apte à:
i) reconnaître ladite caractéristique de forme propre parmi les impulsions détectées sur la liaison bifilaire, de manière à discriminer entre impulsions de stimulation et microimpulsion de pilotage ; et
ii) sur détection d'une microimpulsion de pilotage, commander l'ouverture des moyens interrupteurs (40, 44) de manière à isoler l'(les) électrode(s) (38, 42) de la liaison bifilaire (34, 36), celle-ci n'étant plus reliée qu'au transducteur (24, 26).

8. Un signal de commande, mis en oeuvre dans un dispositif médical implantable actif selon l'une des revendications 1 à 5 et comprenant au moins une commande du groupe formé par :
l'envoi d'un mot de synchronisation ; l'envoi d'un mot d'identification d'un transducteur émetteur et/ou récepteur ; la fermeture de la communication ; le retour à une configuration par défaut du transducteur ; l'écriture de données dans une mémoire du transducteur ; et la lecture de données depuis une mémoire du transducteur.

9. Le signal de la revendication 8, comprenant en outre un champ d'information, comprenant au moins une information du groupe formé par : un mot de synchronisation ; un mot d'identification du transducteur émetteur et/ou récepteur ; des données contenues dans une mémoire du transducteur ; un accusé de réception suite à une action exécutée sur commande du générateur ; et un code de détection et de correction d'erreur.

## Claims

1. An active implantable medical device, comprising :
- a generator (10) comprising
means for analyzing physiological signals and/or producing of stimulation pulses; and means for sending and receiving digital data ; and
- at least a catheter (12, 14, 16) that on the proximal side is connected to the generator and on the distal side comprises
• at least one electrode (38, 42) adapted to contact environing tissues in order to apply said stimulation pulses and/or collect said physiological signals ; a bifilar connection (34, 36) adapted to connect the electrode(s) to the generator ; and
• at least one collector or actuator type transducer (24, 26) ; and
• a communication connection of digital data, adapted to couple said at least one transducer to the generator through the bifilar connection ;
wherein
- means for sending and receiving digital data from the generator comprise means (46, 48, 50, 54) for producing commands to the transducer,
- the transducer comprises means (62, 64, 66, 70) for receiving, decoding and executing said commands, as well as means (62, 64, 66, 68) for sending information in response to the reception of a specific command among the commands produced by the generator, and
- means for sending and receiving digital data from generator comprise means (56) for receiving and decode said information, said device being **characterized in** the fact that :
- the generator furthermore comprises means for producing a control micropulse before sending said commands to the transducer, wherein said micropulse exhibits at least one proper form characteristic that is different from the stimulation pulses ; and
- the catheter furthermore comprises on the distal side :
• controlled switching means (40, 44) selectively interposed between the electrode(s) (38, 42) and the respective conductors (34, 36) of the bifilar connection (34, 36) ; and
- a control circuit (62, 70) coupled to the bifilar connection (34, 36) adapted to :
i) recognize said proper form characteristic among the pulses detected on the bifilar connection, so as to discriminate between the stimulation pulses and the control micropulse ; and
ii) after detection of a control micropulse, command opening of the switching means (40, 44) so as to isolate the electrode(s) (38, 42) from the bifilar connection (34, 36), the latter being no longer connected to the transducer (24, 26).

2. The device according to claim 1, wherein said form characteristic proper to the control pulse is a characteristic belonging to the group consisting of : the micropulse duration ; the micropulse polarity ; the micropulse amplitude ; and combinations of latter characteristics.

3. The device according to claim 1, wherein the control circuit furthermore comprises means (74, 76) for producing a supply voltage based on the pulses detected on the bifilar connection.

4. The device according to claim 1, wherein said commands are commands belonging to the group consisting of : sending a synchronization word ; sending an identification word of the receiver transducer ; closing the communication ; return to the default configuration of the transducer ; writing-in data into a memory of the transducer ; and reading data from a memory of the transducer.

5. The device according to claim 1, wherein said information are information belonging to the group consisting of : a synchronization word ; an identification word of the sending and/or receiving transducer ; data contained in a memory of the transducer ; an acknowledgment following an action executed after a command from the generator ; and an error detection and correction code.

6. A generator for an active implantable medical device according to one of claims 1 to 5, said generator (10) comprising :
- means for connection to a catheter (12, 14, 16) adapted to be connected on the proximal side to the generator, with a bifilar connection (34, 36) adapted to connect to the generator at least one electrode (38, 42) of the catheter, and a connection for communication of digital data, adapted to couple at least one transducer (24, 26) of the catheter to the generator through the bifilar connection ;
- means for analyzing physiological signals and/or producing of stimulation pulses; and
- means for sending and receiving digital data, comprising :
• means (46, 48, 50, 54) for producing commands to the transducer; and
• means (56) for receiving and decode information received from the transducer in response to the reception of a specific command among said commands ;
generator wherein said means for sending and receiving digital data furthermore comprise :
means for producing a control micropulse before sending said commands to the transducer,
wherein said micropulse exhibits at least one proper form characteristic that is different from the stimulation pulses.

7. A catheter for an active implantable medical device according to one of claims 1 to 5, wherein said catheter (12, 14, 16) comprises :
- on the distal side at least one electrode (38, 42) adapted to contact environing tissues in order to apply said stimulation pulses and/or collect said physiological signals ; and at least one collector or actuator type transducer (24, 26) ;
- on the proximal side means for connection to a generator (10) adapted to be connected to the catheter with a bifilar connection (34, 36) adapted to connect the electrode(s) to the generator, and a connection for communication of digital date, adapted to couple the transducer to the generator through the bifilar connection,
- means (62, 64, 66, 70) for receiving, decoding and executing commands produced by the generator ; and
- means (62, 64, 66, 68) for sending information in response to the reception of a specific command among said commands,
wherein said catheter is **characterized in** the fact that it furthermore comprises on the distal side :
- controlled switching means (40, 44) selectively interposed between the electrode(s) (38, 42) and the respective conductors (34, 36) of the bifilar connection (34, 36) ; and
- a control circuit (62, 70) coupled to the bifilar connection (34, 36), adapted to :
i) recognize said proper form characteristic among the pulses detected on the bifilar connection, so as to discriminate between the stimulation pulses and the control micropulse ; and
ii) after detection of a control micropulse, command opening of the switching means (40, 44) so as to isolate the electrode(s) (38, 42) from the bifilar connection (34, 36), the latter being no longer connected to the transducer (24, 26).

8. A command signal implemented in an active implantable medical device according to one of claims 1 to 5, comprising at least one command belonging to the group consisting of : sending a synchronization word : sending of an identification word of a sender and/or receiver transducer : closing the communication ; return to the default configuration of the transducer ; writing-in data into a memory of the transducer ; and reading data from a memory of the transducer.

9. The signal according to claim 8, furthermore comprising an information field comprising at least one information belonging to the group consisting of synchronization word ; an identification word of the sending and/or receiving transducer ; data contained in a memory of the transducer ; an acknowledgment following an action executed after a command from the generator ; and an error detection and correction code.

## Patentansprüche

1. Ein aktives implantierbares medizinisches Gerät, umfassend :
- einen Generator (10) umfassend :
• Mittel zur Analyse von physiologischen Signalen und/oder zur Erzeugung von Stimulationsimpulsen ; und
• Mittel zum Senden und Empfang von numerischen Daten, und
- wenigstens einen auf dem proximalen Ende an den Generator verbundenen Katheter (12,14,16) und umfassend auf dem distalen Ende
• wenigstens eine Elektrode (38, 42), die geeignet ist, in Kontakt mit den umliegenden Geweben zu kommen, um genannte Stimulationsumpulse anzulegen und/oder genannte physiologischen Signale aufzunehmen ;
• eine bifilare Verbindung (34, 36), die geeignet ist, die Elektrode(n) mit dem Generator zu verbinden ; und
• wenigstens einen Wandler (24,26) vom Typ Aufnehmer oder Steller ; und
• eine Verbindung zur Kommunikation von numerischen Daten, die geeignet ist, obengenannten wenigstens einen Wandler über die bifilare Verbindung an den Generator anzukoppeln ;
wobei
- die Mittel zum Senden und Empfang von numerischen Daten des Generators Mittel (46, 48, 50, 54) umfassen, um Befehle an den Wandler zu erzeugen,
- der Wandler Mittel (62, 64, 66, 70) umfasst, um obengenannte Befehle au empfangen, zu dekodieren und auszuführen, sowie Mittel (62, 64, 66, 68) zum Senden Informationen in Beantwortung des Empfangs eines spezifischen Befehls unter den durch den Generator erzeugten Befehlen, und
- die Mittel zum Senden und Empfang von numerischen Daten des Generators Mittel (56) umfassen, um genannte Informationen zu empfangen und zu dekodieren,
**dadurch gekennzeichnet, dass**
- der Generator ausserdem Mittel zur Erzeugung eines Steuerungsmikroimpulses vor dem Senden genannter Befehle an den Wandler umfasst, wobei dieser Mikroimpuls wenigstens eine von den Stimulationsimpulsen verschiedene Eigenformcharakteristik aufweist ; und
- der Katheter auf dem distalen Ende
• gesteuerte, selektiv zwischen der (den) Elektrode(n) (38, 42) and den respektiven Leitern (34, 36) der bifilaren Verbindung (34, 36) zwischengeschaltete Schaltermittel (40, 44) ; und
• einen an die bifilare Verbindung (34, 36) angekoppelten Steuerungsstromkreis, der geeignet ist :
i) obengenannte Eigenformcharakteristik unter den auf der bifilaren Verbindung detektierten Impulsen zu erkennen, um zwischen Stimulastionsimpulsen und Steuerungsmikroimpulsen zu diskriminieren ; und
ii) nach der Detektion einer Steuerungsimpulses das Öffnen der Schaltermittel (40, 44) zu steuern, um die Elektrode(n) (38,42) der bifilaren Verbindung (34, 36) zu isolieren,
wobei letztere nunmehr nur mit dem Wandler (24, 26) verbunden ist,
umfasst.

2. Das Gerät gemäss Anspruch 1, in welchem genannte Eigenformcharakteristik des Mikroimpulses eine Charakteristik ist der Gruppe umfassend die Dauer des Mikroimpulses ; die Polarität des Mikroimpulses ; die Amplitude des Mikroimpulses, und die Kombination genannter Charakteristiken.

3. Das Gerät gemäss Anspruch 1, in welcher der Steuerungsstromkreis ausserdem Mittel (74, 76) zum Erzeugen einer Speisespannung basierend auf die auf der bifilaren Verbindung detektierten Impulse umfasst.

4. Das Gerät gemäss Anspruch 1, in welchem genannte Befehle Befehle sind der Gruppe umfassend die Sendung eines Synchronisationsworts ; die Sendung eines Identifikationsworts des Empfängerwandlers ; das Schliessen der Kommunikation ; die Rückkehr zur Default-Konfiguration des Wandlers ; das Einschreiben der Daten in einen Speicher des Wandlers ; und das Lesen der Daten aus einem Speicher des Wandlers.

5. Das Gerät gemäss Anspruch 1, in welchem genannte Informationen Informationen sind der Gruppe umfassend ein Synchonisationswort ; ein Identifikationswort des Sender- und/oder Empfangswandlers ; in einem Speicher des Wandlers enthaltene Daten ; eine Quittierung nach einer wegen eines Befehls des Generators ausgeführten Tätigkeit ; und einen Detektions- und Fehlerkorrekturcode.

6. Ein Generator für ein aktives implantierbares medizinisches Gerät gemäss einer der Ansprüche 1-5, wobei genannter Generator (10)
- Mittel zur Verbindung mit einem Katheter (12, 14, 16), die geeignet sind, auf dem proximalen Ende mit dem Generator verbunden zu sein, mit einer bifilaren Verbindung (34, 36), die geeignet ist, mit dem Generator wenigstens eine Elektrode (38, 42) des Katheters zu verbinden, mit einer Verbindung zur Kommunikation von numerischen Daten, die geeignet ist, wenigstens einen Wandler (24, 26) des Katheters an den Generator über die bifilare Verbindung anzukoppeln ;
- Mittel zur Analyse von physiologischen Signalen und/oder zur Erzeugung von Stimulationsimpulsen; und
- Mittel zum Senden und Empfang von numerischen Daten, umfassend
• Mittel (46, 48, 50, 54) zur Erzeugung von Befehlen an den Wandler ; und
• Mittel (56) zum Empfang und zur Dekodierung von vom Wandler in Beantwortung des Empfangs eines spezifischen Befehls unter genannten Befehlen empfangenen Informationen ;
umfasst,
wobei genannte Mittel zum Senden und Empfang von numerischen Daten des Generators ausserdem
• Mittel zur Erzeugung eines Steuerungsmikroimpulses vor dem Senden genannter Befehle an den Wandler, wobei genannter Mikroimpuls wenigstens eine von den Steuerungsimpulsen verschiedene Eigenformcharacteristik aufweist ;
umfassen.

7. Ein Katheter für ein aktives implantierbares medizinisches Gerät gemäss einer der Ansprüche 1-5, wobei genannter Katheter (12, 14, 16)
- auf dem distalen Ende wenigstens eine Elektrode (38, 42), die geeignet ist, in Kontakt mit den umliegenden Geweben zu kommen, um Stimulationsimpulse anzulegen und/oder physiologische Signale aufzunehmen ; und wenigstens einen Wandler (24, 26) vom Typ Aufnehmer oder Steller :
- auf dem proximalen Ende Mittel zur Verbindung mit einem Generator (10), die geeignet sind, mit dem Katheter verbunden zu sein, mit einer bifilaren Verbindung (34, 36), die geeignet ist, die Elektrode(n) mit dem Generator zu verbinden, und mit einer Verbindung zur Kommunikation von numerischen Daten, die geeignet ist, den Wandler an den Generator über die bifilare Verbindung anzukoppeln,
- Mittel (62, 64, 66, 70), um durch den Generator erzeugte Befehle zu empfangen, zu dekodieren und auszuführen; und
- Mittel (62, 64, 66, 68) zum Senden von Informationen in Beantwortung des Empfangs eines spezifischen Befehls untergenannten Befehlen ;
umfasst,
wobei genannter Katheter ausserdem auf dem distalen Ende
- gesteuerte, selektiv zwischen der (den) Elektrode(n) (38, 42) und den respektiven Leitern (34, 36) der bifilaren Verbindung (34, 36) zwischengelagerte Schaltermittel (40, 44) ; und
- einen an der bifilaren Verbindung (34, 36) angekoppelten Steuerungsstromkreis, der geeignet ist,
• genannte Eigenformcharakteristik unter den auf der bifilaren Verbindung detektierten Impulsen zu erkennen, um zwischen Stimulationsimpulsen und Steuerungsmikroimpulsen zu diskriminieren ; und
• nach der Detektion einer Steuerungsimpulses das Öffnen der Schaltermittel (40, 44) zu steuern, um die Elektrode(n) (38,42) der bifilaren Verbindung (34, 36) zu isolieren, wobei letztere nunmehr nur mit dem Wandler (24, 26) verbunden ist ; umfasst.

8. Ein in einem aktiven implantierbaren medizinischen Gerät ausgeführtes Steuersignal gemäss einer der Ansprüche 1-5, umfassend wenigstens einen Befehl der Gruppe umfassend die Sendung eines Synchronisationsworts ; die Sendung eines Identifikationsworts eines Sender- und/oder Empfängerwandlers ; das Schliessen der Kommunikation ; die Rückkehr zu einer Default-Konfiguration des Wandlers ; das Einschreiben der Daten in einen Speicher des Wandlers ; und das Lesen der Daten aus einem Speicher des Wandlers.

9. Das Signal gemäss Anspruch 8, ausserdem umfassend ein Informationsfeld umfassend wenigstens eine Information der Gruppe umfassend ein Synchonisationswort ; ein Identifikationswort des Sender- und/oder Empfangswandlers ; in einem Speicher des Wandlers enthaltene Daten ; eine Quittierung nach einer wegen eines Befehls des Generators ausgeführten Tätigkeit ; und einen Detektions- und Fehlerkorrekturcode
